Europäisches Patentamt

European Patent Office

Office européen des brevets

(19)

(11) Veröffentlichungsnummer : **0 396 022 B1**

(12) **EUROPÄISCHE PATENTSCHRIFT**

(45) Veröffentlichungstag der Patentschrift :
03.06.92 Patentblatt 92/23

(21) Anmeldenummer : 90107974.9

(22) Anmeldetag : 26.04.90

(51) Int. Cl.⁵ : **A61K 35/16**

(54) **Verfahren zur Isolierung des Blutgerinnungsfaktors IX.**

(30) Priorität : **05.05.89 DE 3914869**

(43) Veröffentlichungstag der Anmeldung :
**07.11.90 Patentblatt 90/45**

(45) Bekanntmachung des Hinweises auf die
Patenterteilung :
**03.06.92 Patentblatt 92/23**

(84) Benannte Vertragsstaaten :
**AT BE CH DE DK ES FR GB GR IT LI LU NL SE**

(56) Entgegenhaltungen :
**EP-A- 0 041 174**
**EP-A- 0 208 215**
**EP-A- 0 354 354**
**WO-A-83/03760**
**FR-A- 2 234 312**
**US-A- 4 725 673**

(73) Patentinhaber : **BIOTEST PHARMA GMBH**
**Landsteiner Strasse 5**
**W-6072 Dreieich (DE)**

(72) Erfinder : **Möller, Wolfgang, Dr., Dipl.-Chem.**
**Graf-von-Stauffenberg-Strasse 32**
**W-6370 Oberursel (DE)**
Erfinder : **Kraus, Michael, Dipl.-Biol.**
**Fallerslebenstrasse 21**
**W-6000 Frankfurt am Main 1 (DE)**
Erfinder : **Eichentopf, Bertram, Dr.**
**Rosserstrasse 13**
**W-6232 Bad Soden (DE)**

(74) Vertreter : **Beil, Hans Christoph, Dr. et al**
**Beil, Wolff und Beil Rechtsanwälte**
**Adelonstrasse 58**
**W-6230 Frankfurt am Main 80 (DE)**

## Beschreibung

Die Erfindung betrifft ein einfaches Verfahren zur Isolierung des Blutgerinnungsfaktors IX in nahezu reiner Form mit hoher Ausbeute aus Plasma oder anderen Fraktionen durch Adsorption des Faktor IX an eine α-Hydroxyl-Aminogruppen tragende Matrix, Elution des Faktors und der anschliessenden Adsorption an eine ein sulfatiertes Kohlehydrat tragende Matrix.

Nach der Hämophilie A ist die Hämophilie B die zweithäufigste vererbbare Blutgerinnungsstörung. Den Patienten fehlt der für die Gerinnung notwendige Faktor IX. Durch Substitution mit Faktor IX-haltigen Präparationen lässt sich diese vererbbare Gerinnungsstörung behandeln.

Die Behandlung der Hämophilie B wird heute meistens mit den sogenannten PPSB-Konzentraten, die neben dem Faktor IX noch unterschiedliche Mengen der anderen Vitamin K-abhängigen Blutgerinnungsfaktoren II, VII und X enthalten, durchgeführt. Die spezifische Aktivität solcher Präparationen, wie sie z.B. in den EP-B 56 629 und 41 174 beschrieben werden, liegt bei rund 2,5 E Faktor IX pro mg Protein.

Eine Alternative für die PPSB-Konzentrate stellen die hochangereicherten Faktor IX-Konzentrate für die Therapie von Gerinnungsstörungen dar. Hochangereicherte Faktor IX-Konzentrate mit einer spezifischen Aktivität für Faktor IX von mehr als 10 E pro mg Protein lassen sich nach dem heutigen Stand der Technik nur durch mehrstufige Fraktionierungen aus Plasma gewinnen. Beispiele hierfür sind in der Internationalen Patentanmeldung WO 83/03760 oder in DE-A 24 29 191 beschrieben.

In der DE-B 38 26 792 ist die Isolierung eines Faktor IX-Konzentrates mit einer spezifischen Aktivität von bis zu 20 E Faktor IX pro mg Protein mit α-Hydroxyl-Amino-Gruppen tragenden Adsorbentien beschrieben.

In der Arbeit von Michalski, C. et al (Vox Sang. 55 (4), 202-10, 1989) wird ein Verfahren vorgestellt, mit dem sich durch kombinierte Anwendung eines DEAE-Anionenaustauschers und Heparin-Sepharose® ein Faktor IX-Konzentrat mit einer spezifischen Aktivität von rund 10 E pro mg Protein isolieren lässt.

Eine noch höhere Reinheit von 120 bis 150 E Faktor IX pro mg Protein erzielen Burnouf, T. et al. (Int.Symp.on Biotechnology of Plasma Proteins, Nancy 17.05.1988, Abstr.4.09) durch die Anwendung einer zweifachen Anionenaustausch-Chromatographie und einer Affinitäts-Chromatographie. In US-PBS 3 920 625, 4 721 572 und 4 725 673 ist die Hochreinigung von Faktor IX-Konzentraten mit sulfatierten Kohlehydraten, immobilisiert an organische und anorganische Träger, beschrieben. Bei dieser Affinitätschromatographie handelt es sich meist um den letzten Schritt des mehrstufigen Reinigungsverfahrens mit niedriger Gesamtausbeute, bezogen auf das Ausgangsplasma, oder das Endprodukt enthält noch andere Proteine als Verunreinigung. Etwas höhere Ausbeuten wurden oft durch Zusatz von Proteaseinhibitoren, wie z.B. Benzamidin oder PMSF, erreicht. Für therapeutische Anwendungen am Menschen sollte man aber auf den Zusatz von Fremdsubstanzen zum Plasma oder dem Endprodukt möglichst verzichten.

Der vorliegenden Erfindung lag die Aufgabe zugrunde, ein einfaches Verfahren zur Gewinnung einer möglichst reinen Faktor IX-Zubereitung mit einer spezifischen Aktivität von mehr als 150 E Faktor IX pro mg Protein, die weitgehend frei von Fremdproteinen ist, bereitzustellen.

Diese Aufgabe wurde erfindungsgemäss dadurch gelöst, dass man Plasma oder eine andere den Gerinnungsfaktor IX enthaltende Fraktion mit einer die α-Hydroxyl-Amino-Gruppe tragenden Matrix versetzt und den Faktor IX adsorbiert, mit geeigneten Puffern nachwäscht und anschliessend spezifisch eluiert. Der Faktor IX in diesem Eluat wird dann unter Bedingungen an Träger mit immobilisierten sulfatierten Kohlehydraten gebunden, unter denen fast alle anderen Proteine nicht adsorbiert werden. Mit einem Salz-Gradienten wird schliesslich der Faktor IX eluiert.

Überraschenderweise wurde gefunden, dass sich der Faktor IX mit diesem einfachen zweistufigen Verfahren in hoher Ausbeute und in nahezu reiner Form gewinnen lässt. Ein Zusatz von unphysiologischen Proteaseinhibitoren zum Erhalt der Gerinnungsaktivität des Faktor IX war nicht notwendig.

Als Ausgangsmaterial können sowohl Plasma als auch Plasmafraktionen oder andere den Gerinnungsfaktor IX enthaltende Lösungen eingesetzt werden.

Bei dem erfindungsgemässen Verfahren wird z.B. Plasma bei leicht erhöhter Konduktivität, vorzugsweise 12 bis 15 mS/cm, und einem pH von vorzugsweise 7,0 bis 7,7 an einen α-Hydroxyl-Amino-Träger, z.B. Fraktogel® TSK-Amino (Firma Merck, Darmstadt) adsorbiert. Der Träger wird unter Bedingungen gewaschen, unter denen der Faktor IX nur unwesentlich eluiert wird. Dies wird vorzugsweise mit niedermolekularen organischen Aminen, die eine gewisse Ähnlichkeit mit der α-Hydroxyl-Amino-Propyl-Gruppe haben sollten, wie z.B. 1-Amino-2-Propanol, 1,3-Diamino-2-Propanol oder 1,3-Diaminopropan in einem Puffer mit z.B. 10 mM Citrat, 10 mM Phosphat und 100 mM NaCl bei pH 7,5 durchgeführt. Der Waschschritt kann auch mit dem Puffer ohne Amin erfolgen. Allerdings resultiert daraus eine etwas geringere Reinheit und Ausbeute. Durch Erhöhung der Amin- oder gegebenenfalls der Salzkonzentration wird der Faktor IX dann von dem Träger eluiert. Der Zusatz des niedermolekularen Amins erhöht sowohl die Spezifität bei dem Waschschritt als auch bei der Elution des Faktor IX.

Das Eluat mit der Faktor IX-Aktivität wird anschliessend auf einen Träger mit sulfatierten Kohlehydraten, wie z.B. Heparin-Sepharose® (Firma Pharmacia, Freiburg), Heparin-Fraktogel®-TSK, Heparin-Eupergit® (Firma Röhm, Darmstadt) oder Dextransulfat-Sepharose® aufgetragen. Dalsulfatierte Kohlehydrat tragende Matrix Heparin, ist an Polymeren vom Typ eines Copolymerisates von Methacrylamid, N-Methylen-bis-methacrylamid un Allylglycidylether immobilisiert. Die Konduktivität wird dabei auf 13 bis 17, vorzugsweise 15 mS/cm eingestellt. Auf diese Weise wird weitgehend nur Faktor IX an den Träger adsorbiert, der dann anschliessend durch einen Salz-Gradienten mit z.B. 0 bis 1 M NaCl in nahezu reiner Form von der Matrix eluiert werden kann. Das so erhaltene Faktor IX-Eluat wird durch Dia- und Ultrafiltration konzentriert und anschliessend gefriergetrocknet.

Die Adsorption an den α-Hydroxyl-Amino-Träger und an die sulfatiertes Kohlehydrat tragende Matrix kann als Säulenchromatographie, im Batchverfahren oder an Membranen durchgeführt werden.

Die Ausbeute bei diesem einfachen Verfahren liegt bei bis zu 40% der Ausgangsaktivität im Plasma für einen Faktor IX mit einer spezifischen Aktivität von 200 bis 300 E pro mg Protein. Die Gerinnungsaktivität des Faktor IX wurde dabei mit den kommerziell erhältlichen Testreagenzien der Firma Behring nach der Vorschrift des Herstellers bestimmt. Der Proteingehalt der Fraktionen wurde nach der Mikromethode von Bradford, MM., Anal. Biochem. 72, 248-254 (1976) oder nach der Mikrokjeldahl-Methode mit einem Nitrogen-Detektor nach der Vorschrift des Herstellers (Antec Instruments Inc, Houston, Texas) gemessen.

In dem Endprodukt lässt sich mit der Gel-Elektrophorese nach Reisfeld, RA et al., Nature 195, 281 (1962) und Laemmli, UK. et al., Nature 227, 680 (1970) unter reduzierenden und unter nativen Bedingungen weitgehend nur Protein mit Faktor IX-Mobilität nachweisen.

Solche spezifischen Aktivitäten waren bisher nur durch mehrstufige Verfahren zu erreichen, deren Ausbeuten allerdings deutlich unter den 30-40% liegen, die sich mit dem erfindungsgemässen Verfahren erreichen lassen.

Das erfindungsgemässe Verfahren zur Reinigung des Faktor IX stellt damit einen wesentlichen Fortschritt bei der technischen Gewinnung des Gerinnungsfaktor IX dar. Die Vorteile liegen in einer hohen Ausbeute und einer hohen Reinheit bei einem einfachen und schnellen Verfahren.

Vor oder nach der Adsorption des Faktor IX an die α-Hydroxyl-Amino-Matrix bzw. an die sulfatierte Kohlehydrat-Matrix kann man eine Sterilisation mit β-Propiolacton und/oder UV-Bestrahlung, mit Lösungsmittel/Detergentien oder durch Pasteurisierung mit Stabilisatoren durchführen, um eventuell vorhandene humanpathogene Viren zu inaktivieren.

Die nachstehenden Beispiele erläutern die Erfindung.

## Beispiel 1

Eine 250 ml Säule mit einer α-Hydroxyl-Amino-Propyl tragenden Matrix, bestehend aus einem Copolymerisat von Glycidylmethacrylat, Pentaerythrol-dimethacrylat und Polyvinylalkohol, z.B. Fraktogel® TSK-Amino (Fa. Merck, Darmstadt), wird mit 1500 ml Puffer A (10 mM Citrat, 10 mM NaHPO$_4$, 100 mM NaCl, pH 7,5) equilibriert.

5000 ml Plasma mit einer Konduktivität von 13 mS/cm werden mit einer Flußrate von 300 ml/h aufgetragen und dann mit 1000 ml Puffer A gewaschen. Der Faktor IX wird mit einem linearen Gradienten von 0 bis 0,5 M NaCl in Puffer A eluiert. Die Fraktionen mit einer spezifischen Aktivität über 3 E Faktor IX/mg Protein werden gepoolt.

Eine 500 ml Säule mit Heparin-Sepharose® (Pharmacia, Freiburg) wird mit 1000 ml Puffer A equilibriert. Das Faktor IX-Eluat wird mit Puffer B (10 mM Citrat, pH 7,5) auf eine Konduktivität von 15 mS/cm verdünnt und auf die Säule aufgetragen. Anschliessend wird mit 500 ml Puffer B gewaschen und mit einem Gradienten von 0-1 M NaCl in Puffer B eluiert.

Das Faktor IX-Eluat wird durch Diafiltration und Ultrafiltration konzentriert und gefriergetrocknet. Die Gesamtausbeute für den Faktor IX lag bei 30% der Ausgangsaktivität im Plasma. Die spezifische Aktivität des Faktor IX betrug 270 E Faktor IX/mg Protein.

## Beispiel 2

1000 ml Citrat-Plasma werden analog zum Beispiel 1 chromatographiert. Der Faktor IX wird diesmal von der α-Hydroxyl-Amino-Propyl-Matrix Fraktogel® TSK-Amino (Fa. Merck, Darmstadt) mit dem freien Liganden, dem 1-Amino-2-Propanol, eluiert. Nach dem Waschen der Säule mit Puffer A und mit 0,2M 1-Amino-2-Propanol in Puffer A wird mit 0,25 M 1-Amino-2-Propanol in Puffer A eluiert.

Nach der Chromatographie des Faktor IX-Eluates über die Heparin-Sepharose® analog zu Beispiel 1 lag die spezifische Aktivität des Faktor IX bei 265 E Faktor IX/mg Protein. Die Ausbeute betrug 39% der Ausgangsaktivität im Plasma.

Beispiel 3

1000 ml Plasma werden analog zu Beispiel 1 chromatographiert. Als Heparin-Träger wird ein Polymer vom Typ eines Copolymerisates von Glycidylmethacrylat, Pentaerythrol-dimethacrylat und Polyvinylalkohol (Fraktogel®-TKS der Firma Merck, Darmstadt) eingesetzt.

Das Heparin-Fraktogel® wird durch Umsetzung von 50 g Fraktogel®-TSK-Amino mit 1,5 g Heparin und 150 mg $NaCNBH_3$ in 50 ml 0,2 M Phosphatpuffer, pH 7,0, für 2 Tage bei Raumtemperatur und anschliessendem Waschen mit dest. Wasser hergestellt.

Die spezifische Aktivität des Faktor IX lag bei 200 E pro mg Protein bei einer Ausbeute von 27%, bezogen auf das Ausgangsplasma.

Beispiel 4

1000 ml Plasma werden analog zu Beispiel 1 chromatographiert. Anstelle der Heparin-Sepharose® wird Dextransulfat-Sepharose® eingesetzt.

Die Dextransulfat-Sepharose® wird durch Umsetzung von 100 ml Sepharose 4B (Firma Pharmacia, Freiburg) mit 5 g Dextransulfat in 100 ml Wasser und 4 g Bromcyan bei pH 11 für 10 Minuten umgesetzt. Nach weiteren 24 Stunden bei pH 8 wird das Gel mit Wasser und Puffer gewaschen.

Die spezifische Aktivität des Faktor IX lag bei 210 E pro mg Protein bei einer Ausbeute von 31%, bezogen auf das Ausgangsplasma.

Beispiel 5

1000 ml Plasma werden mit 0,25% β-Propiolacton bei pH 8,0 über Nacht behandelt und anschliessend mit UV-Licht bestrahlt. Das kaltsterilisierte Plasma wird analog zu Beispiel 2 aufgearbeitet. Bei einer Ausbeute von 20% lag die Reinheit bei 210 E pro mg Protein.

Beispiel 6

1000 ml Citrat-Plasma werden analog zum Beispiel 2 chromatographiert. Zwischen den beiden Chromatographie-Schritten wird das Eluat des Fraktogel® TSK-Amino für 8 Stunden bei 25°C mit 0,3% Tri-N-butylphosphat und 1% Tween® 80 behandelt und anschliessend in einer Rotationsdurchfluss-Apparatur mit zwei 20 Watt UV-Lampen bei 600 UpM und einem Durchfluss von 20 1 pro Stunde in 1 cm Abstand bestrahlt.

Die spezifische Aktivität des Faktor IX lag bei 245 E Faktor IX/mg Protein. Die Ausbeute betrug 35% der Ausgangsaktivität im Plasma.

Beispiel 7

100 ml eines PPSB-Handelsproduktes mit einer spezifischen Aktivität von 0,6 E Faktor IX/mg Protein werden analog zu Beispiel 2 über je 30 ml der in Beispiel 1 verwendeten Adsorbentien chromatographiert. Auf diese Weise ließ sich ein Faktor IX mit 210 E/mg Protein mit einer Ausbeute von 76% isolieren.

**Patentansprüche**

1. Verfahren zur Isolierung des Blutgerinnungsfaktors IX, dadurch gekennzeichnet, dass man
a) eine den Faktor IX enthaltende Lösung an einem die α-Hydroxyl-Amino-Gruppe tragenden Adsorbens adsorbiert und den Blutgerinnungsfaktor eluiert,
b) den Faktor IX aus dem Faktor IX-Eluat an eine ein sulfatiertes Kohlehydrat tragende Matrix bindet, wobei die Konduktivität auf 13 bis 17 mS/cm eingestellt wird, und anschliessend mit einem Salz-Gradienten eluiert,
c) den reinen Faktor IX konzentriert und gefriertrocknet.

2. Verfahren nach Anspruch 1, dadurch gekennzeichnet, dass man als α-Hydroxyl-Amino-Träger Polymere vom Typ eines Copolymerisates von Glycidylmethacrylat, Pentaerythroldimethacrylat und Polyvinylalkohol einsetzt.

3. Verfahren nach Anspruch 1 bis 2, dadurch gekennzeichnet, dass man als Elutionsmittel für den Faktor IX von dem α-Hyxdroxyl-Amino-Träger ein niedermolekulares primäres organisches Amin verwendet.

4. Verfahren nach Anspruch 3, dadurch gekennzeichnet, dass man als Elutionsmittel 1-Amino-2-Propanol verwendet.

5. Verfahren nach Anspruch 3, dadurch gekennzeichnet, dass man als Elutionsmittel 1,3-Diamino-2-Propanol oder 1,3-Diamino-Propan verwendet.

6. Verfahren nach Anspruch 1 bis 5, dadurch gekennzeichnet, dass man die Adsorption an den α-Hydroxyl-Amino-Träger als Säulenchromatographie, im Batchverfahren oder an Membranen durchführt.

7. Verfahren nach Anspruch 1 bis 6, dadurch gekennzeichnet, dass man als sulfatiertes Kohlehydrat tragende Matrix Heparin, immobilisiert an Polymere vom Typ eines Copolymerisates von Glycidylmethacrylat, Pentaerythrol-dimethacrylat und Polyvinylalkohol, einsetzt.

8. Verfahren nach Anspruch 1 bis 6, dadurch gekennzeichnet, dass man als sulfatiertes Kohlehydrat tragende Matrix Heparin, immobilisiert an Polymere vom Typ eines Copolymerisates von Methacrylamid, N-Methylen-bis-methacrylamid und Allylglycidylether, einsetzt.

9. Verfahren nach Anspruch 1 bis 6, dadurch gekennzeichnet, dass man als sulfatiertes Kohlehydrat tragende Matrix Heparin, immobilisiert an Polymere von Kohlehydraten, einsetzt.

10. Verfahren nach Anspruch 1 bis 6, dadurch gekennzeichnet, dass man als sulfatiertes Kohlehydrat tragende Matrix Dextransulfat, immobilisiert an Polymere von Kohlehydraten, einsetzt.

11. Verfahren nach Anspruch 1 bis 10, dadurch gekennzeichnet, dass man die Adsorption an die sulfatiertes Kohlehydrat tragende Matrix als Säulenchromatographie, im Batchverfahren oder an Membranen durchführt.

12. Verfahren nach Anspruch 1 bis 11, dadurch gekennzeichnet, dass man als Ausgangslösung den Blutgerinnungsfaktor IX enthaltendes Plasma, Plasmafraktionen oder Fermentationslösungen oder Fraktionen davon verwendet.

13. Verfahren nach Anspruch 1 bis 12, dadurch gekennzeichnet, dass man vor oder nach der Adsorption an α-Hydroxyl-Amino-Trägern oder an sulfatierten Kohlehydrat-Trägern in der Lösung vorhandene Viren durch geeignete Massnahmen inaktiviert.

14. Verfahren nach Anspruch 13, dadurch gekennzeichnet, dass man vor oder nach der Adsorption an α-Hydroxyl-Amino-Trägern oder an sulfatierten Kohlehydrat-Trägern in der Lösung vorhandene Viren durch Behandlung mit β-Propiolacton und/oder UV-Bestrahlung, durch Behandlung mit Lösungsmittel und/oder Detergentien oder durch Pasteurisierung mit Stabilisatoren in Lösung oder durch Kombination dieser Verfahren inaktiviert.

## Claims

1. A process for isolating the blood clotting factor IX, characterised in that
a) a solution containing factor IX is adsorbed on adsorbent carrying the α-hydroxyl-amino group and the blood clotting factor is eluted,
b) Factor IX from the Factor IX eluate is bound to a matrix carrying a sulphated carbohydrate, the conductivity being adjusted to from 13 to 17 mS/cm, and Factor IX is subsequently eluted with a salt gradient, and
c) the pure Factor IX is concentrated and freeze dried.

2. A process according to Claim 1, characterised in that the α-hydroxyl-amino carrier used consists of polymers of the type of a copolymer of glycidyl methacrylate, pentaerythritol dimethacrylate and polyvinyl alcohol.

3. A process according to Claims 1 to 2, characterised in that the eluting agent used for eluting Factor IX from the α-hydroxyl-amino carrier is a low molecular weight primary organic amine.

4. A process according to Claim 3, characterised in that the eluting agent used is 1-amino-2-propanol.

5. A process according to Claim 3, characterised in that the eluting agent used is 1,3-diamino-2-propanol or 1,3-diamino-propane.

6. A process according to Claims 1 to 5, characterised in that adsorption on the α-hydroxyl-amino carrier is carried out as column chromatography, by the batch process or on membranes.

7. A process according to Claims 1 to 6, characterised in that the matrix carrying sulphated carbohydrate used is heparin immobilised on polymers of the type of a copolymer of glycidyl methacrylate, pentaerythritol dimethacrylate and polyvinyl alcohol.

8. A process according to Claims 1 to 6, characterised in that the matrix carrying sulphated carbohydrate used is heparin immobilised on polymers of the type of a copolymer of methacrylamide, N-methylene-bis-methacrylamide and allyl glycidyl ether.

9. A process according to Claims 1 to 6, characterised in that the matrix carrying sulphated carbohydrate used is heparin immobilised on polymers of carbohydrates.

10. A process according to Claims 1 to 6, characterised in that the matrix carrying sulphated carbohydrate used is dextran sulphate immobilised on polymers of carbohydrates.

11. A process according to Claims 1 to 10, characterised in that the adsorption on the matrix carrying sulphated carbohydrate is carried out as column chromatography, in the batch process or on membranes.

12. A process according to Claims 1 to 11, characterised in that the starting solution used is plasma, plasma fractions or fermentation solutions or fractions thereof containing blood clotting factor IX.

13. A process according to Claims 1 to 12, characterised in that viruses present in the solution are inactivated by suitable measures before or after the adsorption on $\alpha$-hydroxyl-amino carriers or on sulphated carbohydrate carriers.

14. A process according to Claim 13, characterised in that viruses present in the solution are inactivated by treatment with $\beta$-propiolactone and/or UV irradiation, by treatment with solvents and/or detergents or by pasteurisation with stabilizers in solution or by a combination of these processes before or after the adsorption on $\alpha$-hydroxyl-amino carriers or on sulphated carbohydrate carriers.

## Revendications

1. Procédé d'isolement du facteur de coagulation IX, caractérisé en ce que
a) on adsorbe une solution contenant le facteur IX sur un adsorbant portant le groupe $\alpha$-hydroxy-amino et on élue le facteur de coagulation du sang,
b) on lie le facteur IX provenant de l'éluat du facteur IX sur une matrice portant un hydrate de carbone sulfaté, la conductivité étant réglée à 13 à 17 mS/cm, puis on élue avec un gradient de sel,
c) on concentre le facteur IX pur et on le lyophilise.

2. Procédé selon la revendication 1, caractérisé en ce qu'on utilise comme support d'$\alpha$-hydroxyl-amino des polymères du type d'un copolymère de méthacrylate de glycidyle, de diméthacrylate de pentaérythritol et d'alcool polyvinylique.

3. Procédé selon la revendication 1 ou 2, caractérisé en ce qu'on utilise comme éluant pour le facteur IX du support d'$\alpha$-hydroxy-amino une amine organique primaire à faible poids moléculaire.

4. Procédé selon la revendication 3, caractérisé en ce qu'on utilise comme éluant le 1-amino-2-propanol.

5. Procédé selon la revendication 3, caractérisé en ce qu'on utilise comme éluant le 1,3-diamino-2-propanol ou le 1,3-diamino-propane.

6. Procédé selon l'une des revendications 1 à 5, caractérisé en ce qu'on conduit l'adsorption sur le support d'$\alpha$-hydroxyl-amino sous la forme d'une chromatographie sur colonne, dans un procédé discontinu ou sur des membranes;

7. Procédé selon l'une des revendications 1 à 6, caractérisé en ce qu'on utilise comme matrice portant un hydrate de carbone sulfaté l'héparine, immobilisée sur des polymères du type d'un copolymère de méthacrylate de glycidyle, de diméthacrylate de pentaérythritol et d'alcool polyvinylique.

8. Procédé selon l'une des revendications 1 à 6, caractérisé en ce qu'on utilise comme matrice portant un hydrate de carbone sulfaté, l'héparine, immobilisée sur des polymères du type d'un copolymère de méthacrylamide, de N-méthylène-bis-méthacrylamide et d'allylglycidyléther.

9. Procédé selon l'une des revendications 1 à 6, caractérisé en ce qu'on utilise comme matrice portant un hydrate de carbone sulfaté l'héparine, immobilisée sur des polymères d'hydrate de carbone.

10. Procédé selon l'une des revendications 1 à 6, caractérisé en ce qu'on utilise comme matrice portant un hydrate de carbone sulfaté le sulfate de dextran, immobilisé sur des polymères d'hydrate de carbone.

11. Procédé selon l'une des revendications 1 à 10, caractérisé en ce qu'on conduit l'adsorption sur la matrice portant un hydrate de carbone sulfaté sous la forme d'une chromatographie sur colonne, dans un procédé discontinu ou sur des membranes.

12. Procédé selon l'une des renvendications 1 à 11, caractérisé en ce qu'on utilise comme solution de départ le plasma contenant le facteur de coagulation du sang IX, des fractions de plasma ou des solutions de fermentation ou certaines de leurs fractions.

13. Procédé selon l'une quelconque des revendications 1 à 12, caractérisée en ce qu'avant ou après l'adsorption sur support d'$\alpha$-hydroxyl-amino ou sur supports d'hydrate de carbone sulfaté, on inactive les virus présents dans la solution par des mesures appropriées.

14. Procédé selon la revendication 13, caractérisé en ce qu'avant ou après l'adsorption sur des supports d'$\alpha$-hydroxyl-amino ou sur des supports d'hydrate de carbone sulfaté on inactive les virus présents dans la solution avec de la $\beta$-propiolactone et/ou des radiations UV, par traitement avec des solvants et/ou des détergents ou par pasteurisation avec des stabilisateurs en solution ou par une combinaison de ces procédés.